# EUROPEAN PATENT SPECIFICATION

(11) **EP 3 612 136 B1**
(45) Date of publication and mention of the grant of the patent: **12.06.2024**
(21) Application number: 18723139.4
(22) Date of filing: 18.04.2018
(51) Int. Cl.: A61F 2/24, A61F 2/962, A61M 25/00, A61M 25/01

(54) **CATHETER-BASED DELIVERY DEVICE HAVING SEGMENT WITH NON-UNIFORM WIDTH HELICAL SPINE**
FREISETZUNGSVORRICHTUNG AUF KATHETERBASIS MIT SEGMENT MIT HELIXFÖRMIGEM DORN MIT UNGLEICHFÖRMIGER BREITE
DISPOSITIF D'ADMINISTRATION À BASE DE CATHÉTER AYANT UN SEGMENT AVEC UNE ARMATURE HÉLICOÏDALE À LARGEUR NON UNIFORME

(30) Priority: 19.04.2017 US 201715491200
(43) Date of publication of application: 26.02.2020
(73) Proprietor: Medtronic Vascular, Inc., Santa Rosa, CA 95403 (US)
(72) Inventor: KENNY, Gavin, Santa Rosa California 95403 (US); CIOBANU, Constantin, Santa Rosa California 95403 (US); GRIFFIN, Patrick, Santa Rosa California 95403 (US); LARKIN, Edward, Santa Rosa California 95403 (US)
(74) Representative: Zimmermann & Partner Patentanwälte mbB
(86) International application number: PCT/US2018/028143
(87) International publication number: WO 2018/195181

(56) References cited:
- EP-A1- 1 656 963
- WO-A1-2012/158152
- US-A1- 2015 305 867
- US-A1- 2015 342 762
- US-A1- 2017 056 171

## Description

### FIELD OF THE INVENTION

The present invention relates to systems for percutaneous transcatheter delivery and implantation of a prosthesis, such as a stent, a stent-graft or a prosthetic valve having a stent structure. More particularly, the present invention relates to a segment of a catheter-based delivery device with a non-uniform width helical spine for increased torsional strength.

### BACKGROUND OF THE INVENTION

Among medical catheters commonly used to access vascular and other locations within a body and to perform various functions at those locations are medical catheters, or delivery catheters, adapted to deliver and deploy medical devices such as prosthetic heart valves, stent-grafts, and stents to selected targeted sites in the body. Such medical devices typically are releasably carried within a distal region of the delivery catheter in a radially compressed delivery state as the catheter is navigated to and positioned at a target treatment/deployment site. In many cases, such as those involving cardiovascular vessels, the route to the treatment/deployment site may be tortuous and may present conflicting design considerations requiring compromises between dimensions, flexibilities, material selection, operational controls and the like. One such example is presented in connection with transseptal delivery of a prosthetic heart valve to the left atrium through the right side of the heart that includes a venous route from access through the femoral vein, a vascular route that may require multiple bends.

Typically advancement of a delivery catheter within a patient is monitored fluoroscopically to enable a clinician to manipulate the catheter to steer and guide its distal end through the patient's vasculature to the target treatment/deployment site. This tracking requires a distal end of the delivery catheter to be able to navigate safely to the target treatment/deployment site through manipulation of a proximal end by the clinician. Such manipulation may encompass pushing, retraction and torque forces or a combination of all three. It is therefore required for the distal end of the delivery catheter to be able to withstand all these force.

A delivery catheter desirably will have a low profile/small outer diameter to facilitate navigation through tortuous vasculature; however, small outer diameter catheters present various design difficulties resulting from competing considerations, resulting in design trade-offs. For instance, such delivery catheters must be flexible enough to navigate the tortuous vasculature or anatomy of a patient. However, typical constructions of delivery catheters must attempt to balance a requisite flexibility, with axial strength/stiffness (the property that permits the delivery catheter to be pushed and pulled), and torsional strength/stiffness (the property that permits the delivery catheter to be rotated about its longitudinal axis), especially important is to balance these properties in a distal portion of the delivery catheter within which a prosthesis is held in its compressed, delivery state.

There are various types and constructions of heart valve prostheses that have been suggested for use in percutaneous valve replacement procedures utilizing catheter-based delivery device. In general, the heart valve prostheses attempt to replicate the function of the native valve being replaced and thus will include leaflet-like structures. The heart valve prostheses are generally formed by attaching a bio-prosthetic valve with the leaflet-like structures to a stent-like frame. Such stent-like frames are configured to be radially compressed, or crimped, to enable percutaneous introduction and advancement of the heart valve prosthesis into the vasculature of the patient via a delivery catheter. Once positioned at a desired treatment site, the stent-like frame may be deployed by radially expanding it, or by being formed to be self-expanding, upon release from the catheter-based delivery device.

Prior to release of such a heart valve prosthesis at a treatment site, it may be desirable to adjust a position of the prosthesis in relation to the anatomy of the native valve, such as a native mitral valve, in order to align features of the prosthesis with the anatomy that may be necessary for anchoring and/or assuring proper orientation, and thus functioning, of the prosthesis. However, adjustment of a radial position of the prosthesis relative to a treatment site is often difficult due to the properties of a typical delivery catheter. Typically, a distal portion of a delivery catheter has increased flexibility, which reduces its torsional stiffness. As such, rotation of a proximal end of a delivery catheter may not necessarily provide a directly proportional rotation of either a distal portion of the delivery catheter or a heart valve prosthesis disposed therein. Often, a distal portion of a delivery catheter, such as a distal portion of a sheath of a delivery catheter, may effectively twist relative to a remainder of the catheter and therefore a prosthesis held therein may not be able to be properly radially oriented relative to a treatment site. US 2015/0305867 A1 relates to a prosthetic heart valve delivery apparatus.

Accordingly, there remains a need for an improved catheter-based delivery device with a distal portion that provides a required flexibility along with an increased torsional stiffness for more accurate radial positioning of a prosthesis held therein.

### BRIEF SUMMARY OF THE INVENTION

The invention is a catheter based delivery device as defined by claim 1 and a delivery system as defined by claim 9 for transcatheter delivery of a prosthesis comprising the device of claim 1 and a prosthesis. Embodiments hereof relate to catheter-based delivery devices comprising a sheath configured to transfer a rotational force from a proximal end to a distal end thereof. The sheath includes a segment having a tubular body with a plurality of ribs and slots defined therein, from a proximal end to a distal end of the segment, to provided flexibility to the segment, the segment further having at least one spine that extends or wraps in a helical or spiral path about the tubular body from the proximal end to the distal end of the segment. The at least one spine has a width that is non-uniform along a length thereof for providing a torsional stiffness that decreases from the proximal end to the distal end of the segment. The torsional stiffness of the segment of the sheath permits a rotational force to be transferred from the proximal end to the distal end thereof without the segment twisting relative to a remainder of the sheath. In an embodiment the segment of the sheath is a distal segment of the sheath configured to retain a prosthesis in a radially compressed state therein.

Embodiments hereof also relate to delivery systems for transcatheter delivery of a prosthesis. The delivery systems include a catheter-based delivery device and a prosthesis configured to be held in a radially compressed delivery state within the delivery device and configured to return, or to be returned, to an expanded state at a treatment site after deployment from the delivery device. The catheter-based delivery device includes a sheath having a distal segment that consists essentially of a plurality of ribs, a plurality of slots, and at least one helical spine with a non-uniform width, wherein the non-uniform width of the at least one helical spine provides a non-uniform torsional stiffness along a length of the distal segment. The prosthesis is held in its radially compressed delivery state within the distal segment of the sheath, wherein the delivery device is configured such that rotation of the sheath rotates the distal segment, and the prosthesis disposed therein, substantially in unison for proper alignment with an anatomy of the treatment site.

Embodiments hereof also relate to methods of delivering and deploying a prosthesis at a treatment site. The methods may include advancing a delivery system through the vasculature to the treatment site, wherein the delivery system comprises a catheter-based delivery device and a prosthesis configured to be held in a radially compressed delivery state within the delivery device and configured to return, or to be returned, to an expanded state at a treatment site after deployment from the delivery device. The catheter-based delivery device includes a sheath having a distal segment that consists essentially of a plurality of ribs, a plurality of slots, and at least one helical spine with a non-uniform width, wherein the non-uniform width of the at least one helical spine provides a non-uniform torsional stiffness along a length of the distal segment. The prosthesis is held in its radially compressed delivery state within the distal segment of the sheath. The methods may include radially aligning the prosthesis with an anatomy of the treatment site by rotating the delivery device about a longitudinal axis thereof such that torque applied at a proximal end of the sheath is transferred to a distal end of the sheath through the distal segment of the sheath, whereby the distal segment with the prosthesis disposed therein is rotated substantially in unison with a remainder of the sheath. The methods may include, after rotating the delivery device and radially aligning the prosthesis, retracting the distal segment of the sheath to deploy the prosthesis to an expanded deployed state at the treatment site. In a delivery device for use in methods in accordance herewith, a non-uniform width of the at least one helical spine may decrease from a proximal end to a distal end of a distal segment of the sheath, such that a torsional stiffness of the distal segment decreases from the proximal end to the distal end of the distal segment.

### BRIEF DESCRIPTION OF DRAWINGS

The foregoing and other features and advantages of the invention will be apparent from the following description of embodiments hereof as illustrated in the accompanying drawings. The accompanying drawings, which are incorporated herein and form a part of the specification, further serve to explain the principles of the invention and to enable a person skilled in the pertinent art to make and use the invention. The drawings are not to scale.
FIG. 1 is a side view of an exemplary heart valve prosthesis in an expanded deployed state for use with a catheter-based delivery system in accordance with an embodiment hereof.
FIG. 2 is a side view of a catheter-based delivery system in accordance with an embodiment hereof.
FIG. 3 is an exploded view of the catheter-based delivery system of FIG. 2.
FIGS. 3A and 3AA are cross-sectional views taken along line 3a-3a of FIG. 3 in accordance with embodiments hereof.
FIG. 4 is a perspective view of a distal segment of the catheter-based delivery system of FIG. 2 in accordance with an embodiment hereof, wherein the distal segment is removed from the remainder of the catheter-based delivery system for illustrative purposes.
FIG. 4A depicts a flattened pattern of a distal segment in accordance with an embodiment hereof.
FIG. 5 is a perspective view of the distal segment of FIG. 4, wherein a rotational force is applied at a first end and transmitted to a second end thereof.
FIG. 6A is an end view of the first end of the distal segment of FIG. 4 and FIG. 6B is an end view of the second end of the distal segment of FIG. 4, wherein the rotational force of FIG. 5 is applied at the first end and transmitted to the second end.
FIG. 7 is a perspective view of a distal segment of a catheter-based delivery system in accordance with another embodiment hereof, wherein the distal segment is removed from the remainder of the delivery system for illustrative purposes.
FIG. 8 is a perspective view of a distal segment of a catheter-based delivery system in accordance with another embodiment hereof, wherein the distal segment is removed from the remainder of the delivery system for illustrative purposes.
FIG. 9 is a side view of a distal end of the catheter-based delivery system of FIG. 2 shown disposed within a native heart valve, with a rotational force, as depicted in FIG. 5, applied to a proximal end of the delivery system being transmitted to a distal end of the delivery system.
FIGS. 10-13 are schematic illustrations of a method for delivering and deploying a heart valve prosthesis with the catheter-based delivery system of FIG. 2 in accordance with an embodiment hereof.

### DETAILED DESCRIPTION OF THE INVENTION

Specific embodiments of the present invention are now described with reference to the figures, wherein like reference numbers indicate identical or functionally similar elements. The terms "distal" and "proximal", when used in the following description to refer to a sheath, a catheter-based delivery device, or a catheter-based delivery system are with respect to a position or direction relative to the treating clinician. Thus, "distal" and "distally" refer to positions distant from, or in a direction away from the treating clinician, and the terms "proximal" and "proximally" refer to positions near, or in a direction toward the treating clinician. The terms "distal" and "proximal", when used in the following description to refer to a device to be implanted into a vessel, such as a heart valve prosthesis, are used with reference to the direction of blood flow from the heart. Thus, "distal" and "distally" refer to positions in a downstream direction with respect to the direction of blood flow, and the terms "proximal" and "proximally" refer to positions in an upstream direction with respect to the direction of blood flow.

The following detailed description is merely exemplary in nature and is not intended to limit the invention or the application and uses of the invention. Furthermore, there is no intention to be bound by any expressed or implied theory presented in the preceding technical field, background, brief summary, or the following detailed description.

Valve prostheses for use in accordance with and/or as part of the various delivery systems described herein may have any suitable construction for transcatheter delivery. For instance, a prosthetic valve or a heart valve prosthesis for use with a catheter-based delivery system hereof may have prosthetic leaflets of any suitable nature, and may be specifically configured for replacing a native heart valve or a venous valve. Such a prosthetic valve or heart valve prosthesis may include a stent-like structure within which one, two or three prosthetic leaflets are suitably secured.

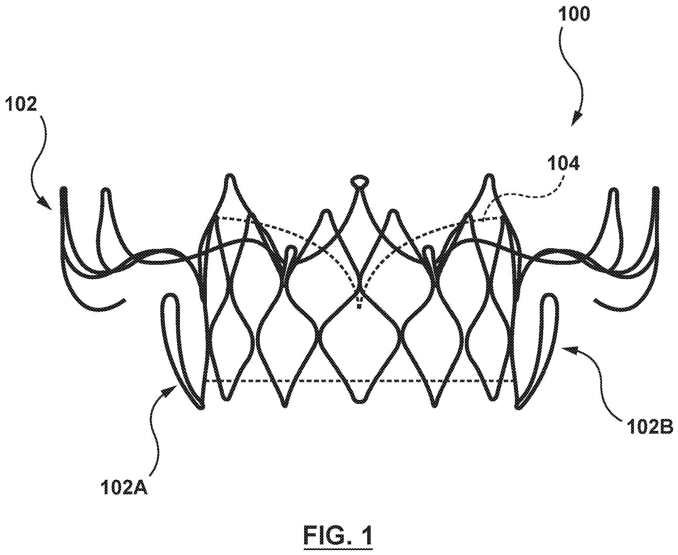

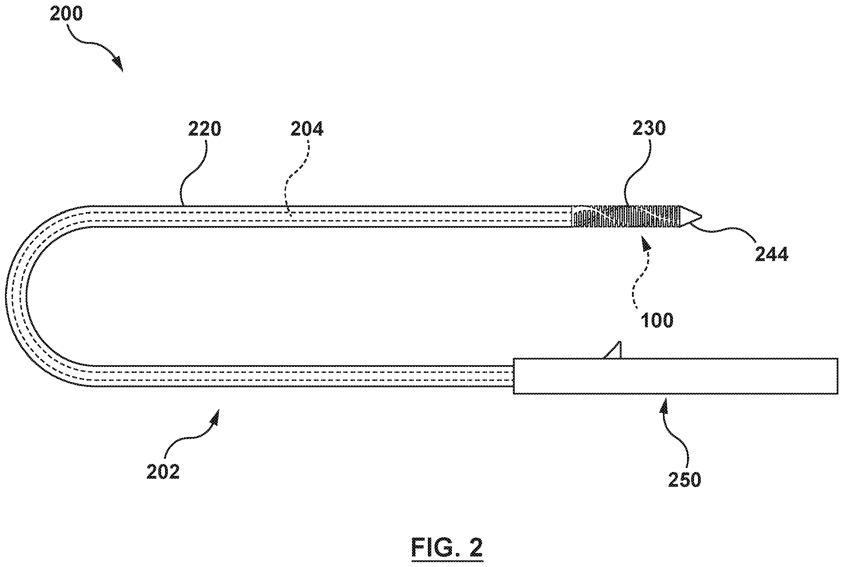

An exemplary heart valve prosthesis 100, which is suitable for use in a catheter-based delivery system in accordance herewith, is shown in FIG. 1. In general terms, the heart valve prosthesis 100 includes a stent-like frame 102 for supporting a valve structure 104, which generally includes 2 to 3 leaflets. In general terms, the stent-like frame 102 is a generally tubular support structure having an internal area or lumen within which a valve structure 104 having leaflets will be secured. The valve structure 104 may be constructed from tissue and/or synthetic materials, as would be known to one of ordinary skill in the art. The heart valve prosthesis 100 has a deployed state as shown in FIG. 1, and a radially compressed, delivery state, as shown in FIG. 2, for loading within a delivery device. The stent-like frame 102 includes a pair of support arms 102A, 102B that are designed to engage native leaflets of a native heart valve when deployed/expanded. Accordingly, the support arms 102A, 102B must be radially aligned at a treatment site in order to properly engage with the anatomy of the native valve. The stent-like frame 102 with the support arms 102a, 102B may be constructed from a shape memory material so as to be configured to self-expand or return to the deployed state of FIG. 1, when released from a delivery device. In embodiments hereof, any of the heart valve prostheses disclosed in U.S. Pat. Appl. Pub. No. 2014/0222142 to Kovalsky et al. and in U.S. Patent No. 8,226,710 to Nguyen et al.*,* may be delivered and deployed by a catheter-based delivery device as described herein.

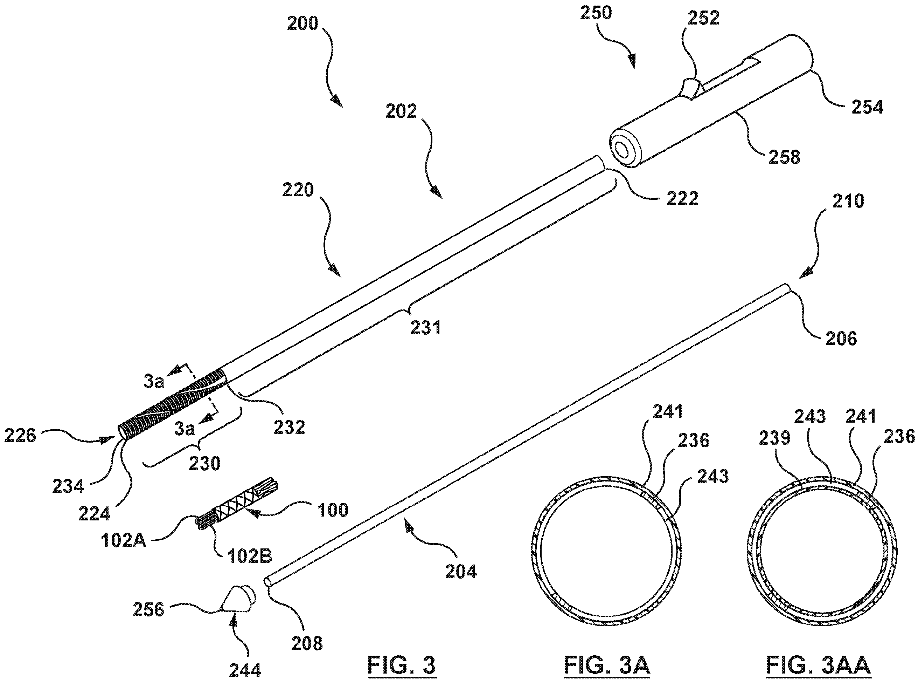

With the above understanding of a suitable valve prosthesis, a delivery system 200 is shown in FIG. 2 and in greater detail in FIG. 3. In an embodiment, the delivery system 200 includes a catheter-based delivery device 202 and a valve prosthesis 100. The valve prosthesis 100 is held in a radially compressed state within a distal segment 230 of a sheath 220 of the delivery device 202. In an embodiment, the delivery system 200 is configured to retain the valve prosthesis 100 in the radially compressed state for delivery to a treatment site of a defective or damaged heart valve. In an embodiment, the delivery system 200 is further configured to release the valve prosthesis at a target deployment site, such as a native mitral valve. It should be understood however that the valve prosthesis 100 is shown by way of example and not limitation and that any other prosthesis may be suitably delivered by the catheter-based delivery device 202 in accordance with embodiments hereof.

In an embodiment in addition to the sheath 220, and with further reference to FIGS. 2-8, the catheter-based delivery device 202 includes an inner shaft 204, which is disposed within the sheath 220, a distal tip component 244, and a handle or hub assembly 250. In embodiments in accordance herewith, the various components of the delivery device 202 recited above may be structures formed from any suitable materials for medical use, such as, but not limited to polyethylene (PE), polyethylene terephthalate (PET), polyvinylchloride (PVC), polyether block amide (PEBAX), Nylon 12, or any other materials suitable for the purposes described herein. Various features of the components of the catheter-based delivery device 202 reflected in FIGS. 3-8 and described below may be modified or replaced with differing structures and/or mechanisms based upon intended application, such as various delivery catheter features described in U.S. Patent No. 8,414,645 to Dwork et al. Accordingly the catheter-based delivery device 202, described in greater detail below, is merely an exemplary embodiment of a transcatheter delivery device according to an embodiment hereof.

In an embodiment, the handle assembly 250 includes a housing 258 and a sheath actuator mechanism 252. The handle assembly 250 is attached to the sheath 220 in a manner that permits the transfer of a rotational force or torque applied thereto to the sheath 220. The handle assembly 250 is shown in FIGS. 2 and 3 with a cylindrical shape, which is by way of example and not limitation as other shapes and sizes may be utilized. For example, in various embodiments, the handle assembly 250 may assume other constructions, such as those described in greater detail in U.S. Patent No. 8,579,963 to Tabor. The sheath actuator mechanism 252 is coupled to the sheath 220, and is generally constructed to provide selective proximal retraction and distal advancement of the sheath 220, and particularly of the distal segment 230, relative to a prosthesis held in a radially compressed, delivery state therein. The sheath actuator mechanism 252 may assume any construction that is capable of providing the desired sheath actuation functionality, such as those described in U.S. Patent No. 8,579,963 to Tabor.

The inner shaft 204 is a tubular component having a proximal end 206, a distal end 208 and a lumen 210 that is defined therebetween. In an embodiment, the lumen 210 may be configured to slidably receive a guidewire therethrough. The inner shaft 204 may be comprised of a single tubular component or of a series of tubular components coupled together. The inner shaft 204 substantially extends between the handle assembly 250 and the distal tip component 244, such that the lumen 210 thereof extends a length of the delivery device 202. As well, the proximal end 206 of the inner shaft 204 is attached to/secured within the handle assembly 250 and the distal end 208 of the inner shaft 204 is attached to/secured within the distal tip component 244. The inner shaft 204 may be coupled to the handle assembly 250 and the distal tip component 244, by way of example and not limitation, by adhesives, welding, clamping, and/or other coupling devices as appropriate. In an embodiment, the proximal end 206 of the inner shaft 204 may be disposed to be accessible at a proximal end 254 of the handle assembly 250 for receiving a guidewire therethrough and the distal end 208 of the inner shaft 204 may be disposed to be accessible at a distal end 256 of the distal tip component 244 for receiving a guidewire therethrough. The inner shaft 204 may assume other constructions, such as those described in greater detail in U.S. Patent No. 8,579,963 to Tabor.

With reference to FIG. 2, the sheath 220 is a generally tubular component having the distal segment 230, as stated above, and a proximal segment 231. The sheath 220 defines a continuous lumen 226 from a proximal end 222 to a distal end 224 thereof. In an embodiment, the sheath 220 is configured to transmit a rotational force or torque received at the proximal end 222 to the distal end 224 thereof. The sheath 220 may be comprised of a single tubular component or of a series of tubular components coupled together, and may be of a single layer or multi-layer construction along its length or any portion of its length. Accordingly, the distal and proximal segments 230, 231 of the sheath 220 may be different segments of a single tubular component, wherein the distal segment 230 has the features as noted below, or may be separate tubular components joined to each other, for example, and not by way of limitation, by fusing, welding, adhesive, and/or other means suitable for the purposes described herein.

The sheath 220 is slidably disposed over the inner shaft 204, and is configured to be longitudinally translated relative to the inner shaft 204 so as to provide selective distal advancement and proximal retraction of the distal segment 230 for covering and uncovering a prosthesis, such as the valve prosthesis 100. The proximal end 222 of the sheath 220 is operably coupled to the sheath actuator mechanism 252 of the handle component 250, such that proximal and distal movement of the sheath actuator mechanism 252 causes the sheath 220 to correspondingly translate relative to the inner shaft 204.

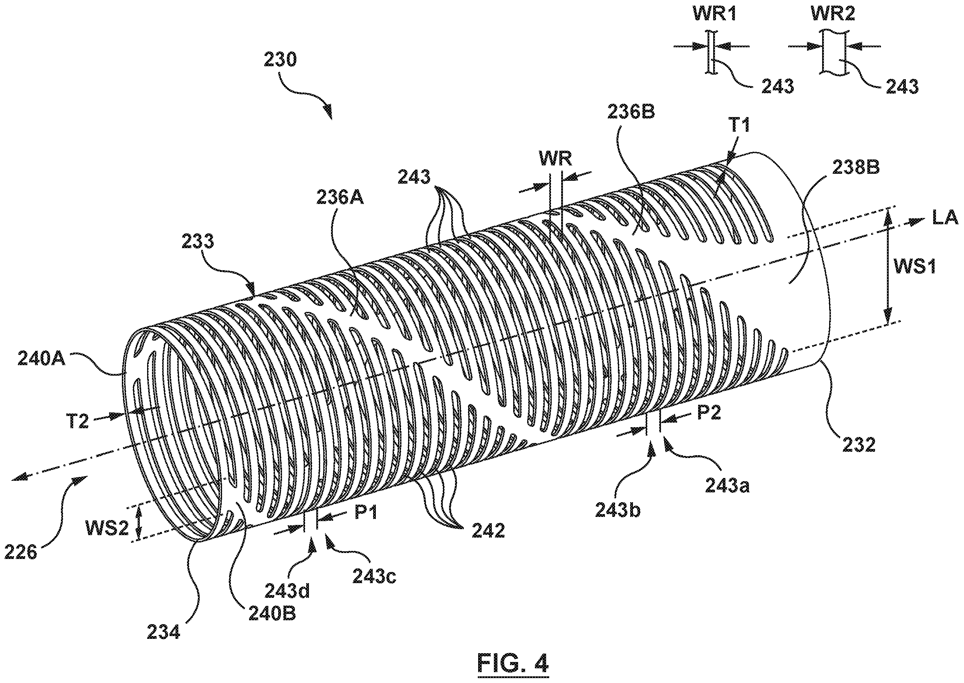

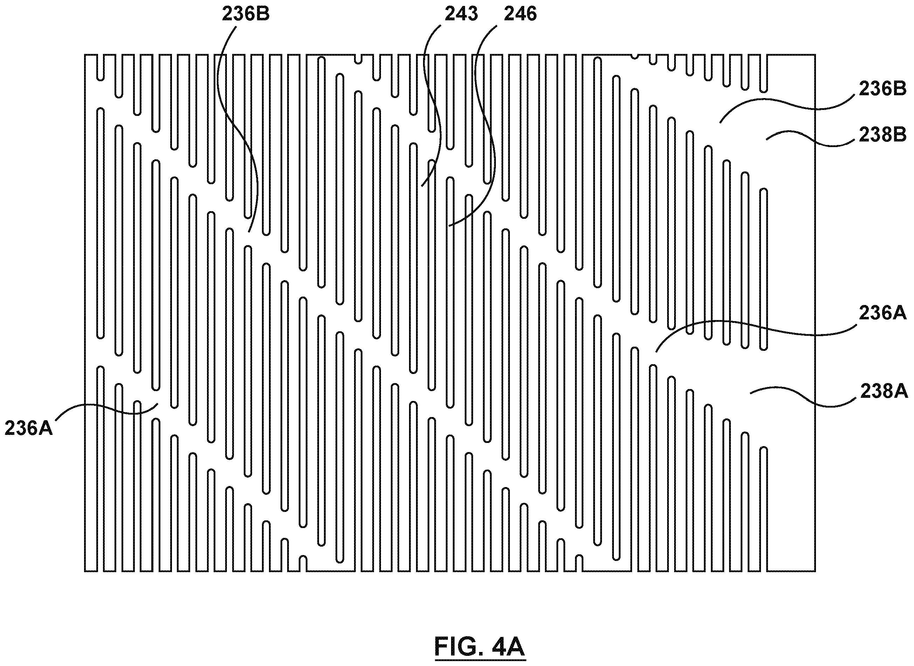

With reference to FIG. 4, the distal segment 230 of the sheath 220 is of a generally tubular shape and includes a proximal end 232 and a distal end 234, and defines a distal portion of the lumen 226 therethrough. In an embodiment, the distal end 234 of the distal segment 230 may be coincident with the distal end 224 of the sheath 220. A tubular body 233 of the distal segment 230 is comprised of a plurality of slots 242 separated, or demarcated, by a plurality of ribs 243, such that generally each rib 243 is separated from an adjacent rib 243 by a slot 242. The tubular body 233 also includes dual spines 236A, 236B that wrap or extend around the tubular body 233 in spiral or helical paths from the proximal end 232 to the distal end 234 of the distal segment 230. Hereinafter, spines 236A, 236B may generally be referred to as helical spines 236A, 236B. The helical spines 236A, 236B intersect with respective ends of each rib 243, and at least partially define respective ends of each slot 242. Stated another way, each rib 243 and each slot 242 circumferentially extends between the helical spine 236A and the helical spine 236B, as shown in FIG. 4A which depicts a flattened pattern of distal segment 233.

In the embodiment of FIG. 4, each helical spine 236A, 236B has a similar right-handed helix, meaning when viewed from the proximal end 232 and distally extending away from a clinician, the spines twist clockwise moving in a distal direction. Due to the similar right-handed helix, the two helical spines 236A, 236B do not intersect along the length of the distal segment 230. However, this is by way of example and not limitation, as one or both of the helical spines 236A, 236B may wrap about a tubular body of a distal segment hereof in a manner of a left-handed helix, and/or may include more or fewer turns. Moreover, in embodiments with more than one helical spine, as shown in the embodiment of FIG. 4, each helical spine may have differing handedness, a differing number of turns, and/or different widths based upon the intended application, and/or the desired properties, for the distal segment 230.

In embodiments hereof, the pattern and/or the shape of the ribs 243 and the slots 242 is configured to provide flexibility to the distal segment 230 of the sheath 220. For example, the flexibility of the distal segment 230 may be increased by utilizing ribs of a thinner width WR1 than a width WR of the ribs in FIG. 4, may be decreased by utilizing ribs of a thicker width WR2 than the width WR of the ribs in FIG. 4, or may be selected to vary along a length of the distal segment by utilizing a combination of thinner and thicker widths WR1, WR2, for example.

In the embodiment of FIG. 4, the slots 242 and the ribs 243 circumferentially extend between the helical spines 236A, 236B in a substantially perpendicular direction from a longitudinal axis LA of the distal segment 230, and are substantially equally spaced from each other along a length of the distal segment 230. Stated another way, the plurality of ribs 243 and the plurality of slots 242 substantially extend in a radial direction that is perpendicular to the longitudinal axis LA of the distal segment 230. The ribs 243 may be considered to have a constant pitch therebetween, or by way of example, a pitch P1 from a center of a first rib 243a to a center of a second, adjacent rib 243b is constant, or equal, to a pitch P2 from a center of a third rib 243c to a center of a fourth, adjacent rib 243d. Essentially, the equal spacing, or pitch, between adjacent ribs 243 along the distal segment 230 provides a constant flexibility there along. In accordance with embodiments hereof, a spacing or pitch may be adjusted, i.e., increased or decreased, in order to provide a desired flexibility for the distal segment. In another embodiment, a spacing or pitch between adjacent ribs 243 may be gradually increased from the proximal end 232 to the distal end 234 of the distal segment 230 in order to provide a gradual increase in flexibility there along. In another embodiment, a width of ribs 243 may be gradually decreased from the proximal end 232 (for e.g., with ribs having a width WR1 as depicted in FIG. 4) to the distal end 234 (for e.g., with ribs having a width WR2 as depicted in FIG. 4) of the distal segment 230 in order to provide a gradual increase in flexibility there along. In another embodiment, a wall thickness of the distal segment 230 may be varied from a thickness T1 at the proximal end 232 to a thickness T2 at the distal end 234 thereof, where T1 >T2, in order to provide a gradual increase in flexibility there along. In an embodiment, a wall thickness of the distal segment 230 may taper from a first thickness T1 at the proximal end 232 to a second, lesser thickness T2 at the distal end 234, such that ribs 243 have different, or varied, wall thickness along a length of the distal segment 230.

In accordance with embodiment hereof, the ribs 243 and the helical spines 236A, 236B are configured to provide radial strength to the distal segment 230 sufficient to retain the valve prosthesis 100 (not shown in FIGS. 4-8) in a radially compressed state during delivery of the prosthesis to a treatment/deployment site. As well, the ribs 243 and the helical spines 236A, 236B are configured to provide axial or columnar strength and torsional stiffness (strength) to the distal segment 230 that balances with the flexibility thereof to permit advancement of the delivery device 202 through the tortuous vasculature of a patient, and to permit proximal retraction of the distal segment 230 for releasing the valve prosthesis 100 from the delivery device 202.

Torsional stiffness (strength) is a desirable property of the distal segment 230 and permits the distal segment 230 to be accurately maneuvered/rotated for proper radial positioning of a prosthesis, such as the valve prosthesis 100, at a desired treatment/deployment site. By torsional stiffness/strength, it is meant that the distal segment 230 of the sheath 220 is configured to transmit a rotational force or torque from the proximal end 232 to the distal end 234 thereof without deformation and/or twisting relative to the proximal segment 231 of the sheath 220.

In the embodiment shown in FIG. 4, axial/columnar strength and torsional stiffness/strength is imparted to the distal segment 230 by the two helical spines 236A, 236B. Each helical spine 236A, 236B follows, or defines, a helical path about the tubular body 233, as noted above, and includes a proximal end 238A, 238B and a distal end 240A, 240B. Each helical spine 236A, 236B has a width that varies, i.e., is non-uniform or not constant, from the proximal end 238A, 238B to the distal end 240A, 240B thereof. The varied or non-uniform width of each helical spine 236A, 236B provides a torsional stiffness (strength) that varies or is non-uniform, as well as a torsional stiffness transition, from the proximal end 232 to the distal end 234 of the distal segment 230 of the sheath 220. In an embodiment, the non-uniform width of each helical spine 236A, 236B decreases from a proximal end to a distal end thereof, such that a width along the length of each helical spine 236A, 236B gradually decreases, or tapers, from a first width WS1 at proximal ends 238A, 238B thereof to a second width WS2 at distal ends 240A, 240B thereof, wherein WS1 >WS2. The non-uniform width provides the distal segment 230 with a non-uniform torsional stiffness along a length L thereof, with a higher torsional stiffness and reduced stress at the proximal end 232 and a lower torsional stiffness at the distal end 234. In other embodiments, the ribs, slots and spine(s) of a distal segment in accordance herewith may be modified to provide a desired flexibility, axial strength, and/or torsional stiffness (strength).

**The** tubular body 233 of the distal segment 230 of the sheath 220 may be formed, by way of example and not limitation, from a tubular component of a metal, such as nitinol, stainless steel, and a Cobalt Chrome (CoCr) alloy, polymers with structural additives (e.g. glass-filled ABS), or a polymer, such as polyetheretherketone (Peek), polyetherimide (PEI), and polyphenylsulfone (PPSU). The ribs 243, slots 242 and spines 236A, 236B of the distal segment 230 may be formed in the tubular body 233, by way of example and not limitation, by machining, laser cutting, 3D printing and/or any other method suitable for the purposes described herein. The proximal end 232 of the distal segment 230 may be fixedly attached by any suitable method to a distal end of the proximal segment 231 so as to be longitudinally translatable relative to the handle component 250 and the inner shaft 204 by the sheath actuator mechanism 252.

FIGS. 3A and 3AA are cross-sectional views taken along line 3a-3a of FIG. 3 in accordance with embodiments hereof. In the embodiment of FIG. 3A, the distal segment 230 is shown as a composite structure having, for e.g., a tubular body 233 that comprises the ribs, slots and spine structures described above with an outer layer 241 of a flexible polymer, such as an elastomeric polymer, for e.g., a thermoplastic urethane. In the embodiment of FIG. 3AA, the distal segment 230 is shown as a composite structure having, for e.g., a tubular body 233 that comprises the ribs, slots and spine structures described above that is sandwiched between an inner layer 239 and an outer layer 241 of a flexible polymer, such as an elastomeric polymer, for e.g., a thermoplastic urethane. In embodiments hereof, the flexible polymer layer(s) may be attached to a tubular body 233, for e.g., by a thermal process, an adhesive, and/or other means suitable for the purposes described herein.

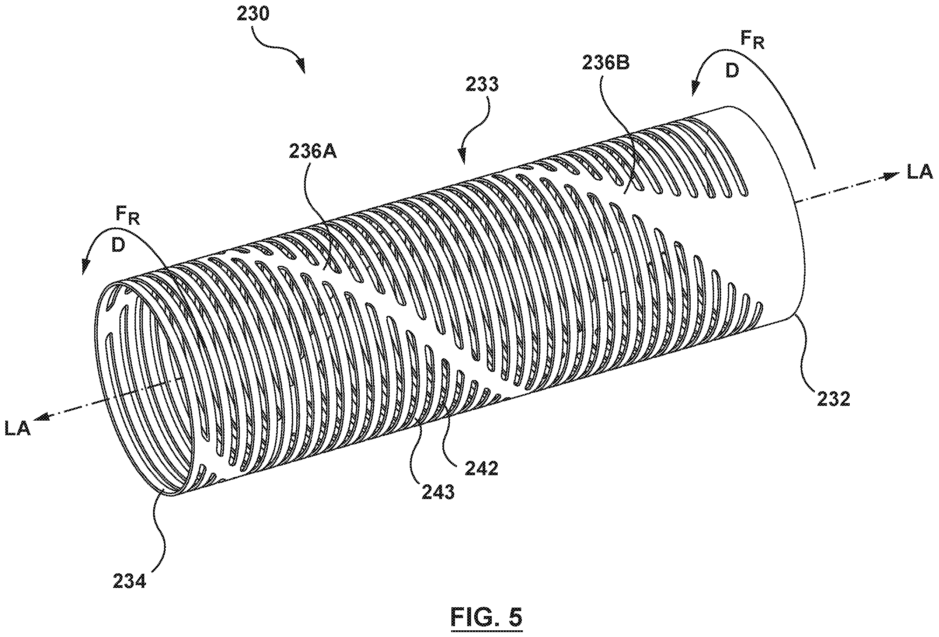

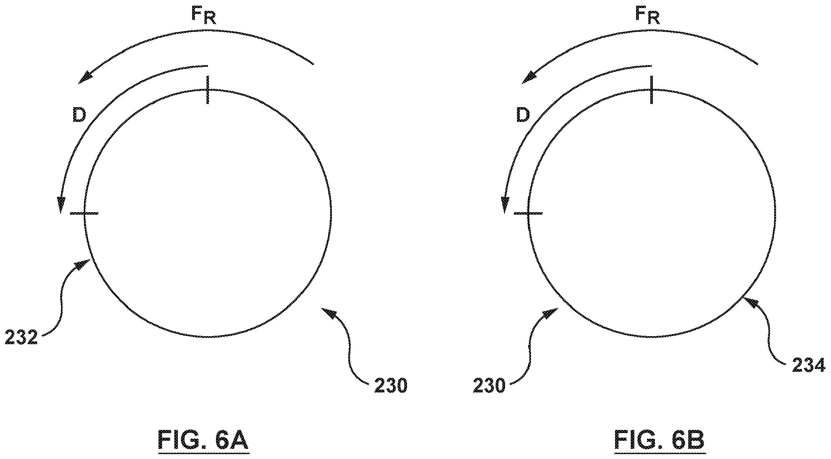

With an understanding of the components of the catheter-based delivery device 202, the interactions of the various components to radially align, and properly deploy, a prosthesis at a treatment site will be described with reference to FIGS. 5, 6A and 6B. In an embodiment, the distal segment 230 of the sheath 220 is configured such that a rotational force or torque F_{R} applied at the proximal end 232 of the distal segment 230 rotates the proximal end 232 a distance D. Due to the torsional stiffness imparted to the distal segment 230 by the first and second spines 236a, 236B, the distal segment 230 transmits the rotational force F_{R} to the distal end 234. The distal segment 230 of the sheath 220 is configured so that it does not twist relative to the proximal segment 231 under the rotational force F_{R}. Thus, the distance D that the proximal end 232 is rotated is substantially equal to the distance D that the distal end 234 is rotated, as depicted in FIGS. 6A and 6B. Stated another way, as represented in FIGS. 5, 6A and 6B, when the proximal end 232 of the distal segment 230 is rotated the distance D by the rotational force F_{R}, the distal end 234 of the distal segment 230 is correspondingly rotated by the distance D by the rotational force F_{R}. In this way, a radial position or alignment of a prosthesis disposed within the distal segment 230 of the sheath 220, such as the valve prosthesis 100 shown in FIG. 3, may be accurately adjusted to assure proper radial alignment with an anatomy of a treatment site.

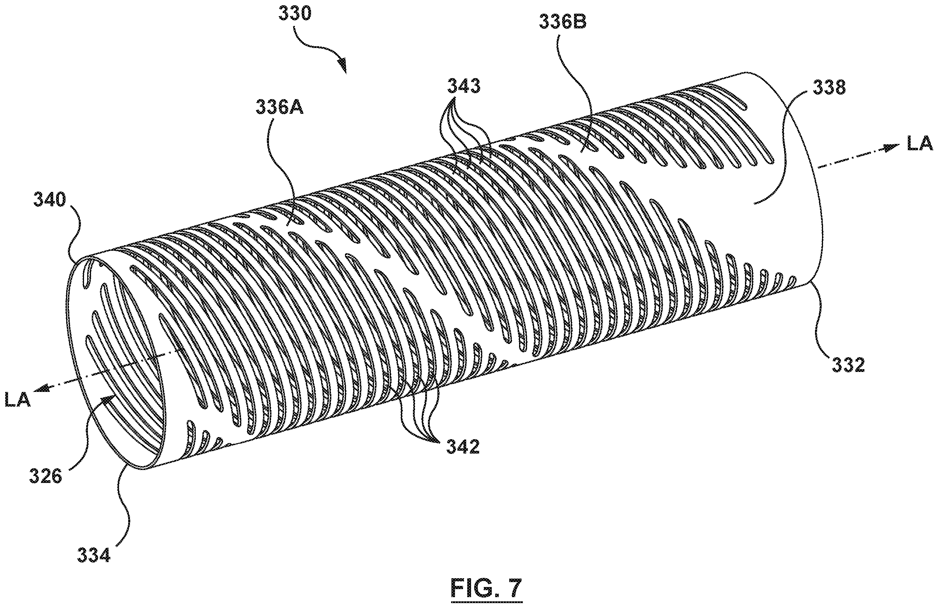

In another embodiment shown in FIG. 7, a distal segment 330 of a sheath or other tubular component includes a proximal end 332 and a distal end 334. The distal segment 330 is similar in all manner to the distal segment 230 except as described herein. The distal segment 330 includes a plurality of ribs 343 (generally known as ribs 343) and a plurality of slots 342 (generally known as slots 342) that are formed to extend between helical spines 336A, 336B. However, instead of extending substantially perpendicular to a longitudinal axis LA of the distal segment 330, the ribs 343 and slots 342 are at other than a right angle with respect to the longitudinal axis LA thereof, such as at an acute angle, for e.g., of 30, 45 or 60 degrees. Stated another way, the plurality of ribs 343 and the plurality of slots 342 substantially extend in a radial direction that is at an acute angle to the longitudinal axis LA of the distal segment 330. Such an arrangement of angled ribs may provide increased structural integrity to the distal segment and/or provide distal segment diameter stability during torqueing.

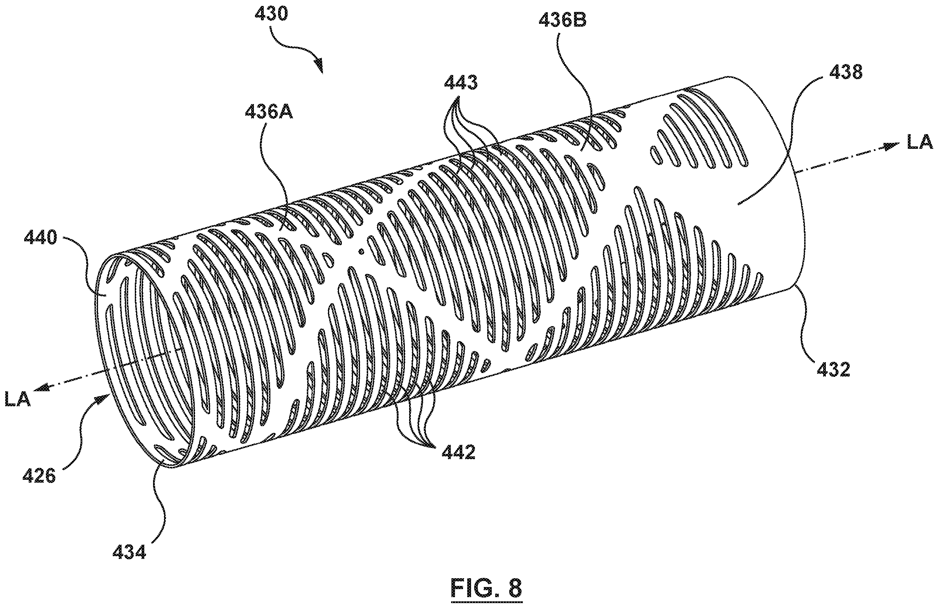

In another embodiment shown in FIG. 8, a distal segment 430 of a sheath or other tubular component includes a proximal end 432 and a distal end 434. The distal segment 430 is similar in all manner to the distal segment 230 except as described herein. Generally, the distal segment 430 includes a plurality of ribs 443 and a plurality of slots 442 and two helical spines 436A and 436B. The first helical spine 436A defines a path that wraps around the distal segment 430 in a manner of a right-handed helix and the second helical spine 436B defines a path that wraps around the distal segment 430 in a manner of a left-handed helix. The first helical spine 436A and the second helical spine 436B so formed spiral opposite each other about the circumference of the distal segment 430 such that they intersect each other at least once along the length of the distal segment 430. While two helical spines are shown in each of the aforementioned embodiments, more than two helical spines may be present with each helical spine having the same or differing handedness and/or non-uniform width based upon the application.

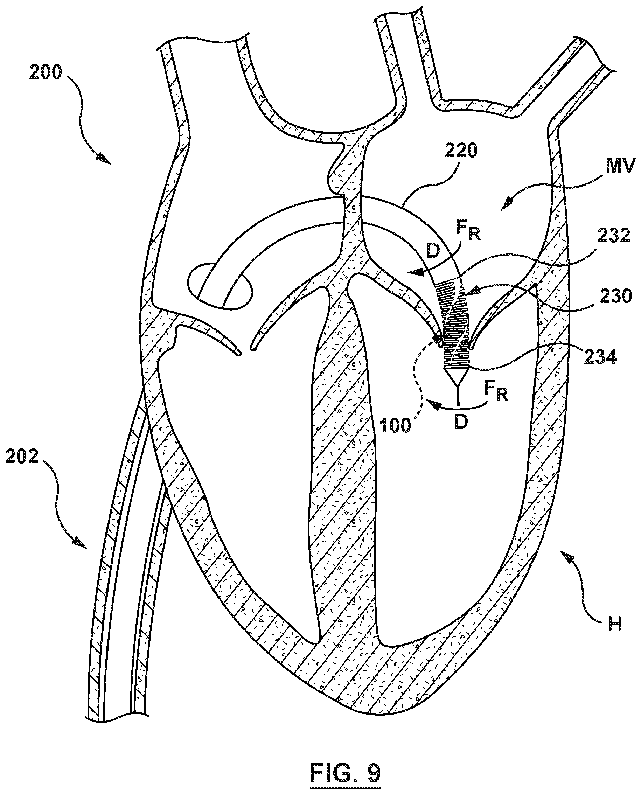

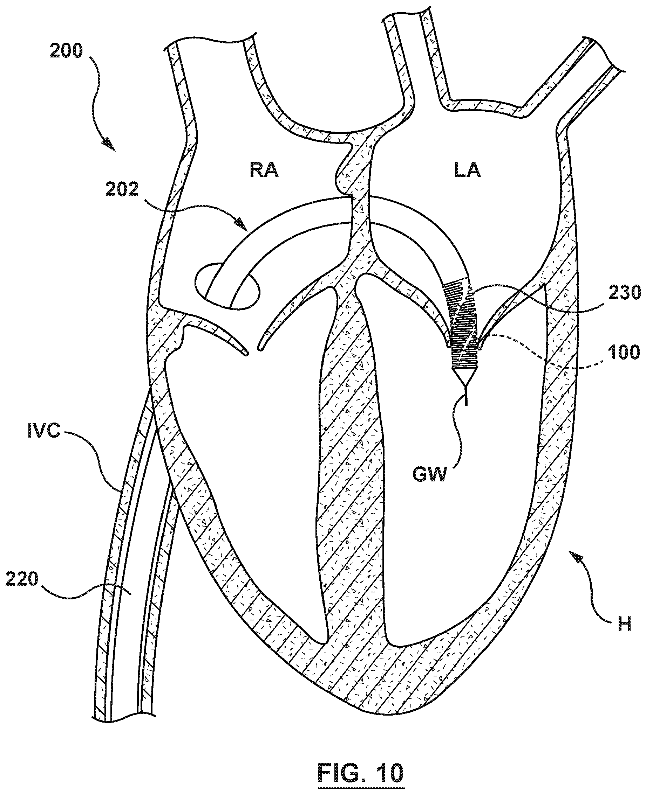

In an embodiment of a method in accordance herewith, the delivery system 200 may be advanced to a treatment site of a mitral valve MV of a heart H via a transseptal approach, as shown in FIG. 9 and explained in more detail with reference to FIGS. 10-13. The distal segment 230 of the sheath 220 may be advanced to extend within/between the leaflets and/or annulus of the mitral valve MV so that the valve prosthesis 100, in this case a mitral valve prosthesis, held in a radially compressed state therein is also positioned within/between the leaflets and/or annulus of the mitral valve MV. In order to properly align the valve prosthesis 100 with the anatomy of the damaged or diseased mitral valve MV, a rotational force or torque may be applied at the handle component 250 (not shown in FIG. 9) that rotates the distal segment 230 of the sheath 220 with the valve prosthesis 100 contained therein. Stated in a simplified manner, a rotational force F_{R} may be applied at the handle component 250 and transferred to the sheath 220, and to the distal segment 230 to effectuate a rotation thereof. The distal segment 230 being configured to have torsional stiffness, as described above due to the non-uniform width helical spines 236A, 236B, does not twist relative to a remainder of the sheath 220 under the rotational force F_{R} but instead substantially transfers the rotational force F_{R} to a distal end 234 of the distal segment 230. In this manner, the distal segment 230 rotates in a unitary fashion substantially the same distance D, from a proximal to a distal end thereof, as depicted in FIG. 9. In this way, a radial position of the valve prosthesis 100 disposed within the distal segment 230 of sheath 220 may be accurately adjusted by a clinician for proper alignment with the anatomy of the treatment/deployment site.

In accordance with an embodiment hereof, FIGS. 10-13 illustrate a method of delivering, positioning and deploying a mitral valve prosthesis via a transseptal approach with a delivery system 200. With reference to FIG. 10, the delivery system 200 is shown after having been introduced into the vasculature via a percutaneous entry point, a.k.a the Seldinger technique, and having been tracked through the vasculature and into the left atrium LA so that the distal segment 230 of the sheath 220 of the catheter-based delivery device 202 is positioned proximate, within or near, the native mitral valve MV. Intravascular advancement to the right atrium RA may be achieved via a percutaneous entry point to a femoral vein and continued advancement through the venous system to the inferior vena cava IVC. Thereafter, a guidewire GW may be advanced along the venous route, directed into and through the right atrium RA to traverse the atrial septum (either by a puncture with the aid of a transseptal needle or via a pre-existing hole therein) and thereby enter the left atrium LA. Once the guidewire GW is so positioned, the endoluminal entry point and the puncture/hole in the atrial septum may be dilated to permit a guide catheter (not shown) to access to the left atrium LA. Thereafter, the delivery system 200 may be advanced through the guide catheter over the indwelling guidewire GW into the left atrium LA and positioned proximate, within or near, the native mitral valve MV. Although described as a transfemoral antegrade approach for percutaneously accessing the native mitral valve, the valve prosthesis 100 may be positioned within the desired area of the heart via the catheter-based delivery device 202 by another method, such as a transseptal antegrade approach via a thoracotomy for accessing the mitral valve, or a transseptal antegrade approach with access via the jugular vein. Although embodiments of the present invention are described with reference to a delivery system for delivering a prosthetic mitral valve, delivery systems in accordance herewith are equally suitable for use in any transcatheter delivered implant potentially requiring rotational alignment, for e.g., transcatheter aortic valve implantation (TAVI) and implantation of a prosthetic valve in a native tricuspid valve. In addition, although described with the use of a guide catheter and a guidewire, in another embodiment hereof the delivery system 200 may access the left atrium without the use of a guidewire and/or a guide catheter.

Further to one of the above-described methods, the catheter-based delivery device 202 including the mitral valve prosthesis 100, which is held in its radially compressed state within the distal segment 230 of the sheath 220, is then positioned at a treatment site of the mitral valve MV, as shown in FIG. 10. In order to be visible under fluoroscopy, the delivery device 202 and the mitral valve prosthesis 100 may also include, for example, radiopaque markers so that a clinician may determine when the delivery device 202, particularly the distal segment 230 thereof, is in a proper location within the native mitral valve MV, and when the mitral valve prosthesis 100 has proper radial alignment for deployment.

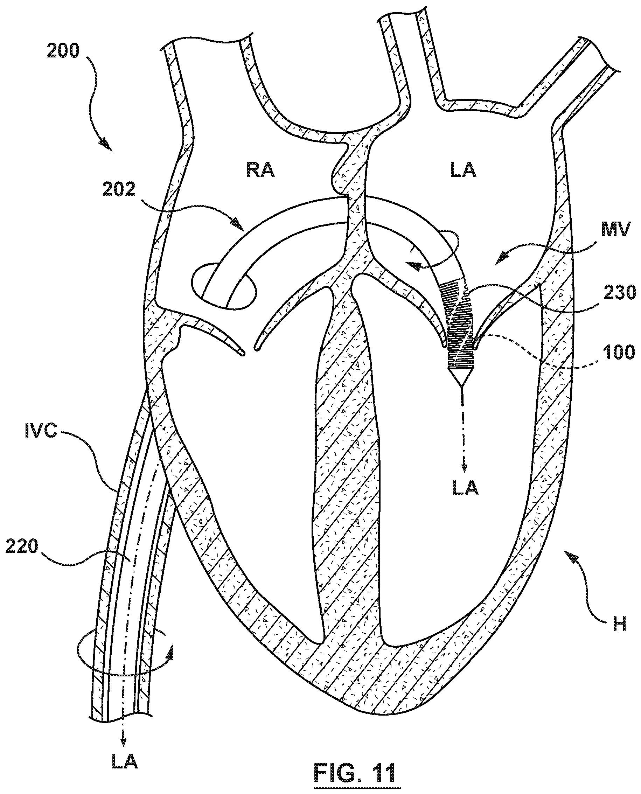

With the catheter-based delivery device 202 at the desired treatment site, if necessary, the delivery device 202 may next be rotated to radially align the mitral valve prosthesis 100 with the anatomy of the native mitral valve MV, for instance to align the supports arms 102A, 102B thereof with the native mitral leaflets. For instance with reference to FIG. 11, under fluoroscopy (or other imaging) it may be determined that the mitral valve prosthesis 100 needs to be rotated in a certain direction by a distance D for proper radial alignment of the support arms 102A, 102B with the leaflets of the native mitral valve MV. Accordingly, a clinician may rotate a handle component (not shown in FIG. 11) of the delivery device 202 about a longitudinal axis LA of the delivery system 200 in the same direction by the desired distance D. Stated another way, application of a rotational force F_{R} to the handle component (not shown in FIG. 11) is transmitted from a proximal end 222 of the sheath 220 to a distal end 224 of the sheath 220 without a loss of torsional force across the distal segment 230 due to the configurations thereof discussed above. Thus, a rotation of the handle component 250 effectuates a substantially equal or similar rotation of the distal segment 230 of the sheath 220 to permit a clinician to make a certain or desired radial alignment of the mitral valve prosthesis 100 at the treatment site.

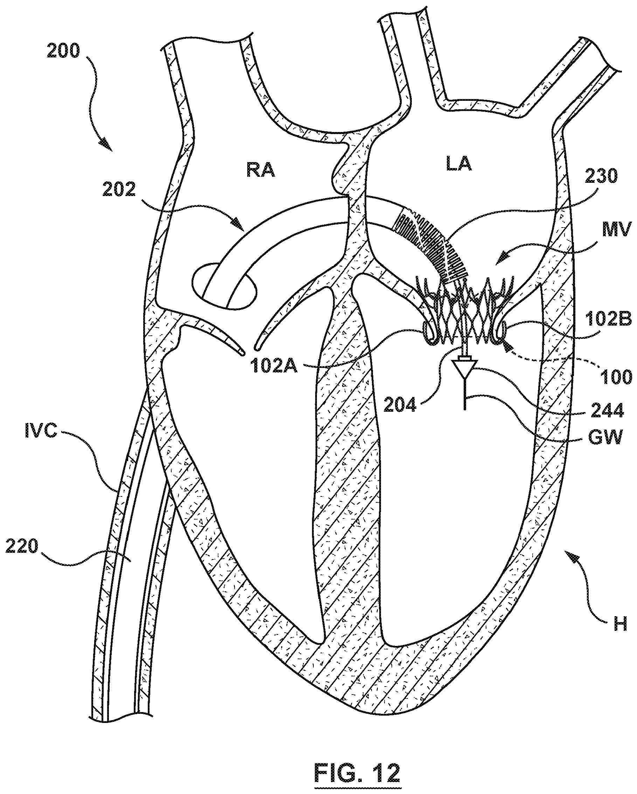

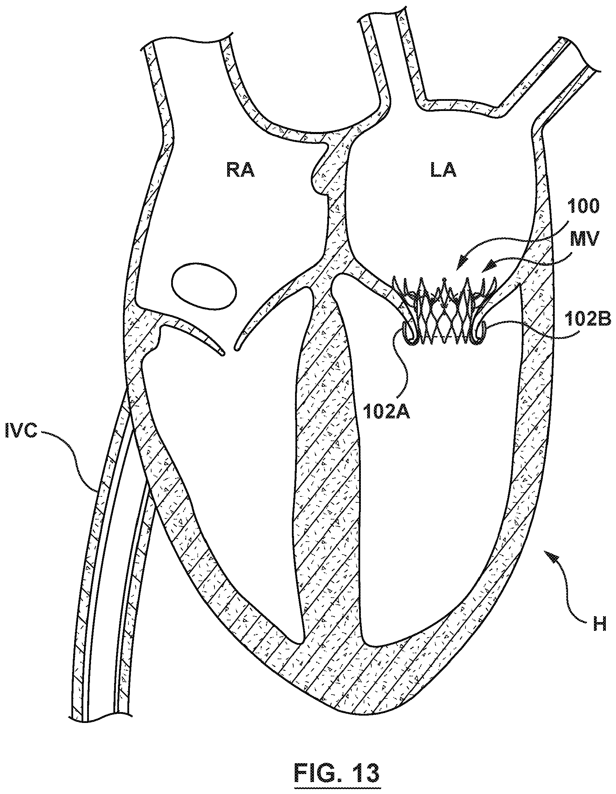

With the mitral valve prosthesis 100 in proper radial alignment within the native mitral valve MV, the sheath 220 with the distal segment 230 may be proximally retracted relative to the inner shaft 204 and the distal tip component 244 to thereby release the mitral valve prosthesis 100, as shown in FIG. 12. The mitral valve prosthesis 100 is then free to self-expand to a radially expanded, deployed state within the native mitral valve MV. Upon radial expansion thereof, the stent-like frame 102 of the mitral valve prosthesis 100 engages with the anatomy of the native mitral valve MV, with the support arms 102A, 102B contacting the leaflets to anchor the prosthesis as shown in FIGS. 12 and 13. Once the mitral valve prosthesis 100 is fully deployed and released from the catheter-based delivery device 202, the delivery device 202 may be retracted and removed from the patient's vasculature leaving the mitral valve prosthesis 100 deployed within the native mitral valve MV, as shown in FIG. 13.

While the method of FIGS. 10-13 illustrate an embodiment of the delivery system 200 with the distal segment portion 230 and the valve prosthesis 100, the methods described in FIGS. 10-13 may be adapted for use with a delivery system having another distal segment and/or prosthesis according to any embodiment described herein. In addition, although the delivery system 200 is shown in FIGS. 10-13 delivering and deploying a valve prosthesis within a native mitral valve via a transseptal approach, this is by way of example and not limitation, as a delivery system in accordance herewith may be used via another approach/pathway for delivering a prosthetic valve to the heart, or other location, within a patient's anatomy. Finally, while the embodiments hereof have been described with reference to a valve prosthesis, and more specifically a mitral valve prosthesis, this is by way of example and not limitation, as embodiments of a delivery system and a delivery device as described herein may be utilized with other deliverable prosthesis, such as, but not limited to, an aortic valve prosthesis, a stent-graft, such as stent-grafts disclosed in U.S. Pat. Appl. Pub. No. 2013/0289702 to Coghlan et al.*,* U.S. Patent No. 8,062,345 to Ouellette et al.*,* U.S. Patent No. 8,882,828 to Kinkade et al.*,* and U.S. Patent No. 9,095,463 to Argentine et al.*,* or other stented prosthesis for use at other locations within a patient's anatomy, such as, but not limited to, a native aortic valve, an aortic aneurysm, or any other body passageway where such may be deemed useful.

While only some embodiments according to the present invention have been described herein, it should be understood that they have been presented by way of illustration and example only, and not limitation. Various changes in form and detail can be made therein without departing from the scope of the invention. Further, each feature of each embodiment discussed herein can be used in combination with the features of any other embodiment.

## Claims

1. A catheter-based delivery device (202) comprising:
a sheath (220) configured to transfer a rotational force from a proximal end (222) to a distal end (224) thereof, the sheath (220) including a distal segment (230, 330, 430) having a tubular body (233) with a plurality of ribs (243, 343, 443) and slots (242, 342, 442) defined therein from a proximal end (232, 332, 432) to a distal end (234, 334, 434) of the distal segment (230, 330, 430) to provide flexibility to the distal segment (230, 330, 430), the distal segment (230, 330, 430) further having at least one spine (236A, 236B, 336A, 336B, 436A, 436B) that extends in a helical or spiral path about the tubular body (233) from the proximal end (232, 332, 432) to the distal end (234, 334, 434) of the distal segment (230, 330, 430),
wherein the at least one spine (236A, 236B, 336A, 336B, 436A, 436B) has a width that is non-uniform along a length thereof for providing a torsional stiffness that decreases from the proximal end (232, 332, 432) to the distal end (234, 334, 434) of the distal segment (230, 330, 430), and
wherein the torsional stiffness of the distal segment (230, 330, 430) permits a rotational force to be transferred from the proximal end (232, 332, 432) to the distal end (234, 334, 434) thereof without the distal segment (230, 330, 430) twisting relative to a remainder of the sheath (220).

2. The catheter-based delivery device (202) of claim 1, wherein the distal segment (230, 330, 430) of the sheath (220) is configured to retain a prosthesis (100) in a radially compressed state therein.

3. The catheter-based delivery device (202) of claim 1, wherein the plurality of ribs (243, 443) and the plurality of slots (242, 442) substantially extend in a radial direction that is perpendicular to a longitudinal axis (LA) of the distal segment (230, 430).

4. The catheter-based delivery device (202) of claim 1, wherein the plurality of ribs (343) and the plurality of slots (342) substantially extend in a radial direction that is at an acute angle to a longitudinal axis (LA) of the distal segment (330).

5. The catheter-based delivery device (202) of claim 1, wherein each rib (243, 243, 443) of the plurality of ribs (243, 243, 443) is separated from an adjacent rib (243, 243, 443) of the plurality of ribs (243, 243, 443) by a slot (242, 342, 442) of the plurality of slots (242, 342, 442).

6. The catheter-based delivery device (202) of claim 1, wherein the width of the at least one spine (236A, 236B, 336A, 336B, 436A, 436B) gradually decreases from a proximal end (238A, 238B, 338, 438) to a distal end of the spine (240A, 240B, 340, 440).

7. The catheter-based delivery device (202) of claim 1, wherein the at least one spine (236A, 236B, 336A, 336B, 436A, 436B) comprises two spines (236A, 236B, 336A, 336B, 436A, 436B) each extending in a helical or spiral path about the tubular body (233) from the proximal end (232, 332, 432) to the distal end (234, 334, 434) of the distal segment (230, 330, 430).

8. The catheter-based delivery device (202) of claim 7, wherein the two spines (436A, 436B) wrap in opposite directions around the tubular body of the distal segment (430) such that the two spines (436A, 436B) intersect each other in at least one location; or
wherein the two helical spines (236A, 236B, 336A, 336B) wrap in a same direction around the tubular body of the distal segment (230, 330) such that the two helical spines (236A, 236B, 336A, 336B) do not intersect each other.

9. A delivery system (200) for transcatheter delivery of a prosthesis comprising:
a catheter-based delivery device (202) according to claim 1,; and
a prosthesis (100) configured to be disposed within the distal segment (230, 330, 430) of the sheath (220) in a radially compressed delivery state and configured to return, or to be returned, to an expanded state after deployment from the distal segment (230, 330, 430) of the sheath (220).

10. The delivery system (200) of claim 9, wherein the prosthesis (100) disposed within the distal segment, substantially in unison for proper alignment with an anatomy of a treatment site.

11. The delivery system (200) of claim 9, wherein the non-uniform width of the at least one helical spine (236A, 236B, 336A, 336B, 436A, 436B) decreases from a proximal end (232, 332, 432) to a distal end (234, 334, 434) of the distal segment (230, 330, 430).

12. The delivery system (200) of claim 9, wherein the at least one helical spine (236A, 236B, 336A, 336B, 436A, 436B) comprises two helical spines (236A, 236B, 336A, 336B, 436A, 436B) each extending in a helical or spiral path about the distal segment (230, 330, 430) from a proximal end (232, 332, 432) to a distal end (234, 334, 434) thereof.

13. The delivery system (200) of claim 12, wherein the two helical spines (436A, 436B) wrap in opposite directions around the distal segment (430) of the sheath (220) such that the two helical spines (436A, 436B) intersect each other in at least one location.

14. The delivery system (200) of claim 12, wherein the two helical spines (236A, 236B, 336A, 336B) wrap in a same direction around the distal segment (230, 330) of the sheath (220) such that the two helical spines (236A, 236B, 336A, 336B) do not intersect each other.

15. The delivery system (200) of claim 9, wherein the prosthesis (100) is a prosthetic valve having a stent-like structure (102); or wherein the prosthesis (100) is a stent-graft.

## Patentansprüche

1. Katheterbasierte Abgabevorrichtung (202), umfassend:
eine Hülle (220), die konfiguriert ist, um eine Drehkraft von einem proximalen Ende (222) auf ein distales Ende (224) davon zu übertragen, wobei die Hülle (220) ein distales Segment (230, 330, 430) einschließt, das einen röhrenförmigen Körper (233) mit einer Vielzahl von Rippen (243, 343, 443) und Schlitzen (242, 342, 442) aufweist, die von einem proximalen Ende (232, 332, 432) zu einem distalen Ende (234, 334, 434) des distalen Segments (230, 330, 430) darin definiert sind, um dem distalen Segment (230, 330, 430) Flexibilität bereitzustellen, wobei das distale Segment (230, 330, 430) ferner mindestens einen Dorn (236A, 236B, 336A, 336B, 436A, 436B) aufweist, der sich in einem wendelförmigen oder spiralförmigen Weg um den röhrenförmigen Körper (233) von dem proximalen Ende (232, 332, 432) zu dem distalen Ende (234, 334, 434) des distalen Segments (230, 330, 430) erstreckt,
wobei der mindestens eine Dorn (236A, 236B, 336A, 336B, 436A, 436B) eine Breite aufweist, die entlang einer Länge davon ungleichmäßig ist zum Bereitstellen einer Torsionssteifigkeit, die von dem proximalen Ende (232, 332, 432) zu dem distalen Ende (234, 334, 434) des distalen Segments (230, 330, 430) abnimmt, und
wobei die Torsionssteifigkeit des distalen Segments (230, 330, 430) es ermöglicht, dass eine Drehkraft von dem proximalen Ende (232, 332, 432) auf das distale Ende (234, 334, 434) davon übertragen wird, ohne dass sich das distale Segment (230, 330, 430) relativ zu einem Rest der Hülle (220) verdreht.

2. Katheterbasierte Abgabevorrichtung (202) nach Anspruch 1, wobei das distale Segment (230, 330, 430) der Hülle (220) konfiguriert ist, um eine Prothese (100) in einem radial komprimierten Zustand darin zu halten.

3. Katheterbasierte Abgabevorrichtung (202) nach Anspruch 1, wobei sich die Vielzahl von Rippen (243, 443) und die Vielzahl von Schlitzen (242, 442) im Wesentlichen in eine radiale Richtung erstrecken, die zu einer Längsachse (LA) des distalen Segments (230, 430) senkrecht ist.

4. Katheterbasierte Abgabevorrichtung (202) nach Anspruch 1, wobei sich die Vielzahl von Rippen (343) und die Vielzahl von Schlitzen (342) im Wesentlichen in eine radiale Richtung erstrecken, die zu einer Längsachse (LA) des distalen Segments (330) in einem spitzen Winkel ist.

5. Katheterbasierte Abgabevorrichtung (202) nach Anspruch 1, wobei jede Rippe (243, 243, 443) der Vielzahl von Rippen (243, 243, 443) von einer angrenzenden Rippe (243, 243, 443) der Vielzahl von Rippen (243, 243, 443) durch einen Schlitz (242, 342, 442) der Vielzahl von Schlitzen (242, 342, 442) getrennt ist.

6. Katheterbasierte Abgabevorrichtung (202) nach Anspruch 1, wobei die Breite des mindestens einen Dorns (236A, 236B, 336A, 336B, 436A, 436B) von einem proximalen Ende (238A, 238B, 338, 438) zu einem distalen Ende des Dorns (240A, 240B, 340, 440) allmählich abnimmt.

7. Katheterbasierte Abgabevorrichtung (202) nach Anspruch 1, wobei der mindestens eine Dorn (236A, 236B, 336A, 336B, 436A, 436B) zwei Dornen (236A, 236B, 336A, 336B, 436A, 436B) umfasst, die sich jeder in einem wendelförmigen oder spiralförmigen Weg um den röhrenförmigen Körper (233) von dem proximalen Ende (232, 332, 432) zu dem distalen Ende (234, 334, 434) des distalen Segments (230, 330, 430) erstrecken.

8. Katheterbasierte Abgabevorrichtung (202) nach Anspruch 7, wobei sich die zwei Dornen (436A, 436B) in entgegengesetzten Richtungen um den röhrenförmigen Körper des distalen Segments (430) derart wickeln, dass die zwei Dornen (436A, 436B) an mindestens einem Ort einander schneiden; oder
wobei sich die zwei wendelförmigen Dornen (236A, 236B, 336A, 336B) in einer gleichen Richtung um den röhrenförmigen Körper des distalen Segments (230, 330) derart wickeln, dass die zwei wendelförmigen Dornen (236A, 236B, 336A, 336B) nicht einander schneiden.

9. Abgabesystem (200) für eine Transkatheterabgabe einer Prothese, umfassend: eine katheterbasierte Abgabevorrichtung (202) nach Anspruch 1; und
eine Prothese (100), die konfiguriert ist, um innerhalb des distalen Segments (230, 330, 430) der Hülle (220) in einem radial komprimierten Abgabezustand angeordnet zu werden, und konfiguriert ist, um nach einem Einsatz aus dem distalen Segment (230, 330, 430) der Hülle (220) in einen expandierten Zustand zurückzukehren oder dahin gebracht zu werden.

10. Abgabesystem (200) nach Anspruch 9, wobei die Prothese (100) innerhalb des distalen Segments angeordnet ist, im Wesentlichen in Einklang für eine richtige Ausrichtung mit einer Anatomie einer Behandlungsstelle.

11. Abgabesystem (200) nach Anspruch 9, wobei die ungleichmäßige Breite des mindestens einen wendelförmigen Dorns (236A, 236B, 336A, 336B, 436A, 436B) von einem proximalen Ende (232, 332, 432) zu einem distalen Ende (234, 334, 434) des distalen Segments (230, 330, 430) abnimmt.

12. Abgabesystem (200) nach Anspruch 9, wobei der mindestens eine wendelförmige Dorn (236A, 236B, 336A, 336B, 436A, 436B) zwei wendelförmige Dornen (236A, 236B, 336A, 336B, 436A, 436B) umfasst, die sich jeder in einem wendelförmigen oder spiralförmigen Weg um das distale Segment (230, 330, 430) von einem proximalen Ende (232, 332, 432) zu einem distalen Ende (234, 334, 434) davon erstrecken.

13. Abgabesystem (200) nach Anspruch 12, wobei sich die zwei wendelförmigen Dornen (436A, 436B) in entgegengesetzten Richtungen um das distale Segment (430) der Hülle (220) derart wickeln, dass die zwei wendelförmigen Dornen (436A, 436B) an mindestens einem Ort einander schneiden.

14. Abgabesystem (200) nach Anspruch 12, wobei sich die zwei wendelförmigen Dornen (236A, 236B, 336A, 336B) in einer gleichen Richtung um das distale Segment (230, 330) der Hülle (220) derart wickeln, dass die zwei wendelförmigen Dornen (236A, 236B, 336A, 336B) nicht einander schneiden.

15. Abgabesystem (200) nach Anspruch 9, wobei die Prothese (100) eine prothetische Klappe ist, die eine stentartige Struktur (102) aufweist; oder wobei die Prothese (100) ein Stent-Implantat ist.

## Revendications

1. Dispositif d'administration par cathéter (202) comprenant :
une gaine (220) conçue pour transférer une force de rotation d'une extrémité proximale (222) à une extrémité distale (224) de celle-ci, la gaine (220) comportant un segment distal (230, 330, 430) ayant un corps tubulaire (233) avec une pluralité de nervures (243, 343, 443) et de fentes (242, 342, 442) définies à l'intérieur de celui-ci, d'une extrémité proximale (232, 332, 432) à une extrémité distale (234, 334, 434) du segment distal (230, 330, 430) pour fournir une flexibilité au segment distal (230, 330, 430), le segment distal (230, 330, 430) ayant en outre au moins une colonne (236A, 236B, 336A, 336B, 436A, 436B) qui s'étend dans une trajectoire hélicoïdale ou en spirale autour du corps tubulaire (233) de l'extrémité proximale (232, 332, 432) à l'extrémité distale (234, 334, 434) du segment distal (230, 330, 430),
dans lequel l'au moins une colonne (236A, 236B, 336A, 336B, 436A, 436B) a une largeur qui n'est pas uniforme sur sa longueur afin de fournir une rigidité en torsion qui diminue de l'extrémité proximale (232, 332, 432) à l'extrémité distale (234, 334, 434) du segment distal (230, 330, 430), et
dans lequel la rigidité en torsion du segment distal (230, 330, 430) permet de transférer une force de rotation de l'extrémité proximale (232, 332, 432) à l'extrémité distale (234, 334, 434) de celui-ci sans que le segment distal (230, 330, 430) ne se torde par rapport au reste de la gaine (220).

2. Dispositif d'administration par cathéter (202) selon la revendication 1, dans lequel le segment distal (230, 330, 430) de la gaine (220) est conçu pour retenir une prothèse (100) dans un état de compression radiale dans celui-ci.

3. Dispositif d'administration par cathéter (202) selon la revendication 1, dans lequel la pluralité de nervures (243, 443) et la pluralité de fentes (242, 442) s'étendent sensiblement dans une direction radiale perpendiculaire à l'axe longitudinal (LA) du segment distal (230, 430).

4. Dispositif d'administration par cathéter (202) selon la revendication 1, dans lequel la pluralité de nervures (343) et la pluralité de fentes (342) s'étendent sensiblement dans une direction radiale qui forme un angle aigu avec l'axe longitudinal (LA) du segment distal (330).

5. Dispositif d'administration par cathéter (202) selon la revendication 1, dans lequel chaque nervure (243, 243, 443) de la pluralité de nervures (243, 243, 443) est séparée d'une nervure adjacente (243, 243, 443) de la pluralité de nervures (243, 243, 443) par une fente (242, 342, 442) de la pluralité de fentes (242, 342, 442).

6. Dispositif d'administration par cathéter (202) selon la revendication 1, dans lequel la largeur de l'au moins une colonne (236A, 236B, 336A, 336B, 436A, 436B) diminue progressivement d'une extrémité proximale (238A, 238B, 338, 438) à une extrémité distale de la colonne (240A, 240B, 340, 440).

7. Dispositif d'administration par cathéter (202) selon la revendication 1, dans lequel l'au moins une colonne (236A, 236B, 336A, 336B, 436A, 436B) comprend deux colonnes (236A, 236B, 336A, 336B, 436A, 436B) s'étendant chacune dans une trajectoire hélicoïdale ou en spirale autour du corps tubulaire (233) de l'extrémité proximale (232, 332, 432) à l'extrémité distale (234, 334, 434) du segment distal (230, 330, 430).

8. Dispositif d'administration par cathéter (202) selon la revendication 7, dans lequel les deux colonnes (436A, 436B) s'enroulent dans des directions opposées autour du corps tubulaire du segment distal (430) de telle sorte que les deux colonnes (436A, 436B) s'entrecroisent à au moins un endroit ; ou
dans lequel les deux colonnes hélicoïdales (236A, 236B, 336A, 336B) s'enroulent dans une même direction autour du corps tubulaire du segment distal (230, 330) de telle sorte que les deux colonnes hélicoïdales (236A, 236B, 336A, 336B) ne s'entrecroisent pas.

9. Système d'administration (200) destiné à l'administration transcathéter d'une prothèse comprenant : un dispositif d'administration par cathéter (202) selon la revendication 1 ; et
une prothèse (100) conçue pour être disposée à l'intérieur du segment distal (230, 330, 430) de la gaine (220) dans un état d'administration comprimée de manière radiale et conçue pour revenir ou être ramenée à un état d'expansion après déploiement à partir du segment distal (230, 330, 430) de la gaine (220).

10. Système d'administration (200) selon la revendication 9, dans lequel la prothèse (100) est disposée dans le segment distal, sensiblement à l'unisson pour un alignement correct sur l'anatomie d'un site de traitement.

11. Système d'administration (200) selon la revendication 9, dans lequel la largeur non uniforme de l'au moins une épine hélicoïdale (236A, 236B, 336A, 336B, 436A, 436B) diminue d'une extrémité proximale (232, 332, 432) à une extrémité distale (234, 334, 434) du segment distal (230, 330, 430).

12. Système d'administration (200) selon la revendication 9, dans lequel l'au moins une colonne hélicoïdale (236A, 236B, 336A, 336B, 436A, 436B) comprend deux colonnes hélicoïdales (236A, 236B, 336A, 336B, 436A, 436B) s'étendant chacune dans une trajectoire hélicoïdale ou en spirale autour du segment distal (230, 330, 430) d'une extrémité proximale (232, 332, 432) à une extrémité distale (234, 334, 434) de ce dernier.

13. Système d'administration (200) selon la revendication 12, dans lequel les deux épines hélicoïdales (436A, 436B) s'enroulent dans des directions opposées autour du segment distal (430) de la gaine (220) de telle sorte que les deux épines hélicoïdales (436A, 436B) s'entrecroisent à au moins un endroit.

14. Système d'administration (200) selon la revendication 12, dans lequel les deux épines hélicoïdales (236A, 236B, 336A, 336B) s'enroulent dans la même direction autour du segment distal (230, 330) de la gaine (220) de telle sorte que les deux épines hélicoïdales (236A, 236B, 336A, 336B) ne s'entrecroisent pas.

15. Système d'administration (200) selon la revendication 9, dans lequel la prothèse (100) est une valve prothétique ayant une structure de type endoprothèse (102) ; ou dans lequel la prothèse (100) est une endoprothèse couverte.
